# FASCICULE DE BREVET EUROPEEN

(11) **EP 2 866 863 B1**
(45) Date de publication et mention de la délivrance du brevet: **29.06.2016**
(21) Numéro de dépôt: 13765451.3
(22) Date de dépôt: 27.06.2013
(51) Int. Cl.: A61M 5/31, A61M 5/315

(54) **DISPOSITIF DE DIFFUSION D'UN PRODUIT LIQUIDE A VISÉE MEDICAMENTEUSE COMPORTANT UN CORPS, UN POUSSOIR ET UN BOUCHON, LA SURFACE DU BOUCHON ET/OU LE CORPS COMPORTANT UN COMPOSÉ HYDROPHILE.**
VORRICHTUNG ZUR ABGABE EINES FLÜSSIGEN MEDIZINPRODUKTS MIT EINEM KÖRPER, EINEM SCHIEBER UND EINER KAPPE MIT EINER OBERFLÄCHE DER KAPPE UND/ODER DES KÖRPERS AUS EINEM HYDROPHILEN BESTANDTEIL
DEVICE FOR DISPENSING A LIQUID MEDICAL PRODUCT, COMPRISING A BODY, A PUSHER AND A CAP, THE SURFACE OF THE CAP AND/OR THE BODY COMPRISING A HYDROPHILIC COMPOUND

(30) Priorité: 27.06.2012 FR 1256128
(43) Date de publication de la demande: 06.05.2015
(73) Titulaire: Association pour les Transferts de Technologies du Mans, 72000 Le Mans (FR)
(72) Inventeur: MARMEY, Pascal, 72000 Le Mans (FR)
(74) Mandataire: Gevers & Orès
(86) Numéro de dépôt international: PCT/IB2013/055283
(87) Numéro de publication internationale: WO 2014/002045

(56) Documents cités:
- WO-A1-2008/151074
- WO-A1-2011/092536
- US-A1- 2008 071 228

## Description

L'invention concerne un dispositif comprenant un corps, un poussoir et un bouchon situé sur une extrémité du poussoir pour assurer l'étanchéité entre le corps et le poussoir.

Plus précisément, l'invention concerne un tel dispositif muni d'un traitement hydrophile pour améliorer le glissement entre le bouchon et le corps.

Il peut s'agir d'une seringue, d'une pompe ou de tout dispositif connu formant un dispositif de diffusion d'un produit fluide, notamment à visée médicamenteuse.

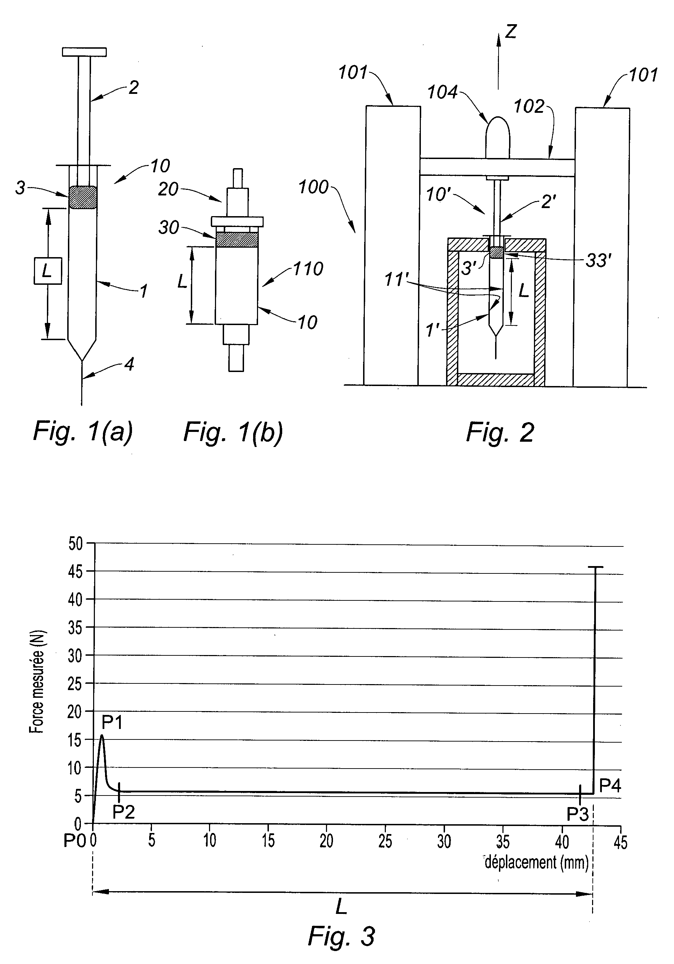

La figure 1 (a) représente une seringue classique.

Cette seringue 10 est munie d'un corps 1 de seringue, d'un poussoir 2 et d'un bouchon 3 dont la fonction est d'assurer l'étanchéité entre le corps 1 de seringue et le poussoir 2. A cet effet, le bouchon 3 est généralement réalisé avec un élastomère. Le bouchon 3 est généralement monté sur une extrémité du poussoir 2.

Cette seringue 10 est également munie d'une aiguille 4, disposée à l'extrémité du corps de seringue pour injecter le produit contenu dans le corps 1 de seringue à une personne ou à un animal ou, a contrario, effectuer un prélèvement sanguin ou autre. Dans d'autres cas, la seringue peut ne pas comporter d'aiguille, mais prévoir à la place des connexions avec, à titre non limitatif, des tubulures, cathéters ou poches. Par exemple, il peut être prévu une connexion de type « Luer ».

Le bouchon 3 doit également présenter de bonnes caractéristiques de glissement.

En effet, lorsqu'un utilisateur injecte un produit ou prélève du sang ou autre, l'utilisation de la seringue est alors plus aisée.

A cet effet, le bouchon 3 est généralement enduit d'huile de silicone.

Cependant, ces huiles de silicone, bien que biocompatibles, pourrait à l'avenir ne pas pouvoir être utilisées. En effet, de plus en plus de produits médicamenteux sont des protéines synthétisées par biotechnologies. Or, de telles protéines, par exemple des anticorps, sont généralement de grosses molécules comportant des blocs hydrophobes peu compatibles avec l'emploi d'huile de silicone (ci-après nommée « silicone »).

WO 2011/092536 décrit un dispositif de diffusion d'un fluide à visée médicamenteuse tel qu'une seringue ou d'autres systèmes d'injection sans aiguille comprenant un corps, un poussoir et un bouchon monté sur une extrémité du poussoir. La surface interne du corps est séparée en deux zones fonctionnelles bien distinctes, chacune de ces zones présentant des états de surface distincts. La première zone peut être enduite d'un produit hydrophile et la seconde zone peut être enduite de produits à base de silicone ou de fluor, donc de produits généralement hydrophobes. Le polymère hydrophile est choisi dans une liste incluant des polysaccharides tels que les polymères d'acide hyaluronique. Le corps est réalisé en verre ou en plastique. Quant au bouchon, il est fait de caoutchouc ou de matériau élastomérique. La figure 1(b) représente quant à elle une pompe utilisée classiquement pour injecter une substance médicamenteuse à une personne par voie naturelle (bouche, nez) ou par injection à travers la peau.

Cette pompe 110 est munie d'un corps 10 de pompe, d'un poussoir/piston 20 et d'un bouchon 30 dont la fonction est d'assurer l'étanchéité entre le corps 10 seringue et le poussoir 20. Dans ce cas, le bouchon 30 appartient au piston 20 et en forme une extrémité.

Les pompes connues sont, comme les seringues, souvent enduites de silicone, généralement au niveau du corps 10 de pompe.

Un objectif de l'invention est ainsi de proposer un dispositif du type précité ne comportant pas de silicone et présentant des caractéristiques de glissement au moins sensiblement similaires à celles d'un dispositif enduit de silicone.

Un autre objectif de l'invention est de proposer un dispositif du type précité avec des caractéristiques de glissement améliorées par rapport à celles du silicone.

En particulier, il convient de noter que le bouchon peut coller à la paroi du corps du dispositif au début de la mise en mouvement du poussoir par rapport au corps.

Dans un tel cas, l'utilisateur a donc tendance à appuyer plus fort sur le poussoir pour débloquer le bouchon (« break-loose » selon la terminologie anglo-saxonne). Par la suite, une injection d'un produit dans la personne ou l'animal à traiter peut s'avérer brutale, voire douloureuse avec certains produits.

Pour cette raison, un objectif plus particulier de l'invention est de proposer un dispositif du type précité qui offre une performance comparable voire limite, par rapport aux dispositifs dans lesquels le bouchon et/ou le corps est enduit de silicone, les risques de blocage du bouchon dans le corps lors de la mise en mouvement du poussoir.

En particulier également, il convient de noter que pendant la course du bouchon dans le corps du dispositif, le glissement du bouchon siliconé dans le corps peut également être imparfait et/ou nécessiter une force de poussée non négligeable.

C'est pourquoi un autre objectif particulier de l'invention est de proposer un dispositif qui présente, par rapport aux dispositifs dont le bouchon et/ou le corps est enduit de silicone, un glissement du bouchon le long du corps du dispositif qui est comparable, voire meilleur.

Pour résoudre l'un au moins de ces problèmes, l'invention propose ainsi un dispositif comprenant au moins un corps, un poussoir et un bouchon situé à l'extrémité du poussoir pour assurer l'étanchéité entre le poussoir et le corps, caractérisé en ce que l'un au moins, d'une part, de l'intégralité de la surface interne du corps ou, d'autre part, d'au moins une partie de la surface externe du bouchon, est enduite d'un produit hydrophile choisi parmi le Hyaluronate de sodium (HANa), la polyvinylpyrrolidone (PVP) de haut poids moléculaire, le polyéthylène glycol (PEG) de haut poids moléculaire ou l'Hydroxypropyl Methyl Cellulose (HPMC).

Ce dispositif pourra également présenter l'une au moins des caractéristiques suivantes, prise seule ou en combinaison :
- l'intégralité de la surface interne du corps et au moins une partie de la surface externe du bouchon sont enduites d'un même produit hydrophile ;
- l'intégralité de la surface externe du bouchon est enduite du produit hydrophile ;
- la polyvinylpyrrolidone de haut poids moléculaire est la PVP K90 ;
- le polyéthylène glycol de haut poids moléculaire est le PEG 900 000 ;
- l'HPMC est l'HPMC E4M ;
- le corps est réalisé en verre ou en polymère tel qu'un copolymère cyclo-oléfine, le polyéthylène ou le polypropylène ;
- le dispositif n'est pas une seringue ;
- le dispositif est une pompe ;
- le dispositif est une seringue ;
- le bouchon est réalisé en élastomère.

L'invention sera mieux comprise et d'autres buts, avantages et caractéristiques de celle-ci apparaîtront plus clairement à la lecture de la description qui suit et qui est faite au regard des dessins annexés, sur lesquels :
- la figure 2 représente un schéma d'une installation expérimentale permettant de caractériser les propriétés mécaniques d'un dispositif conforme à l'invention ;
- la figure 3 représente une courbe théorique de l'effort appliqué sur le poussoir du dispositif, en fonction du déplacement de ce poussoir le long du corps, avec l'installation expérimentale de la figure 2 ;
- la figure 4 représente la force de décollement, mesurée par l'installation expérimentale de la figure 2, du bouchon d'une seringue conforme à l'invention par rapport au corps de la seringue lorsque ce bouchon est mis en mouvement dans le corps de la seringue et ce, pour différentes seringues ;
- la figure 5 représente la force de poussée, mesurée par l'installation expérimentale de la figure 2, qui est exercée sur le poussoir de la seringue lors du mouvement du bouchon le long du corps de la seringue, après décollement et ce, pour les seringues dont la force de décollement est représentée sur la figure 4 ;
- la figure 6 représente la force de décollement, mesurée par l'installation expérimentale de la figure 2, du bouchon de seringue par rapport au corps de la seringue lorsque ce bouchon est mis en mouvement dans le corps de la seringue et ce, pour d'autres seringues ;
- la figure 7 représente la force de poussée, mesurée par l'installation expérimentale de la figure 2, exercée sur le poussoir de la seringue lors du mouvement du bouchon le long du corps de la seringue, après décollement et ce, pour les seringues dont la force de décollement est représentée sur la figure 6 ;
- la figure 8 représente la force de décollement, mesurée avec l'installation expérimentale de la figure 2, du bouchon de seringue par rapport au corps de la seringue lorsque ce bouchon est mis en mouvement dans le corps de la seringue et ce, pour encore d'autres seringues ;
- la figure 9 représente la force de poussée, mesurée par l'installation expérimentale de la figure 2, exercée sur le poussoir de la seringue lors du mouvement du bouchon le long du corps de la seringue, après décollement et ce, pour les seringues dont la force de décollement est représentée sur la figure 8.

La figure 2 est un schéma d'une installation expérimentale permettant de tester différents dispositif conformes à l'invention.

Cette installation expérimentale 100 comprend un dynamomètre, de marque MTS, muni d'un bâti 101 et d'une traverse 102 reposant sur le bâti 101. Cette traverse peut se déplacer selon un axe vertical (axe Z) le long du bâti 101.

Cette installation expérimentale 100 est en l'occurrence représentée combinaison avec une seringue, car les différents tests présentés ci-après ont été réalisés avec une telle seringue.

Le corps 1' de la seringue est mis en place sur un portoir 103, qui permet de bloquer le corps de seringue. Le poussoir 2' est quant à lui monté sur la traverse 102 et peut donc subir un mouvement de translation axial (axe Z) dans le corps 1' de la seringue 10', lorsque la traverse 102 est mise en mouvement le long du bâti 101. L'extrémité du poussoir 2' est munie d'un capteur de force 104, qui permet donc de déterminer la force appliquée sur le poussoir 2'.

L'installation expérimentale telle que représentée sur la figure 2 permet d'effectuer des essais en compression, c'est-à-dire, pour le cas d'une seringue, lorsque le poussoir 2' rentre dans le corps 1' de la seringue 10'.

Avant de tester un dispositif conforme à l'invention sur l'installation expérimentale précitée, le traitement qui suit doit être effectué sur la ou chaque partie destinée à recevoir des molécules visant à remplacer le silicone. Dans le cas d'un dispositif enduit de silicone, c'est généralement le fabricant qui fournit lui-même le dispositif siliconé, si bien qu'un tel traitement n'est pas nécessaire.

Ce traitement préalable est un traitement plasma, réalisé sur une machine Isytech Plasmatreat RF. Les parties du dispositif devant être traitées sont disposées dans une enceinte, munie d'électrodes et d'une entrée pour des gaz réactifs, tels que l'Argon, le diazote ou le dioxygène. Le vide est réalisé dans l'enceinte (0,1mbar). Le plasma est généré par un générateur de haute fréquence fonctionnant à 13,56MHz, dont la puissance maximale est de 600W.

Ce traitement plasma améliore la mouillabilité des parties de la seringue destinées à être enduites par la molécule choisie et ainsi, faciliter l'accrochage de cette molécule sur la partie de la seringue considérée.

En effet, cette molécule est un produit hydrophile.

Comme cela sera détaillé par la suite, les différentes molécules testées sont choisies parmi :
- la famille des Glycosaminoglycanes comme des dérivés de l'acide Hyaluronique tel que le Hyaluronate de sodium (HANa) ou comme l'héparine ;
- des polymères vinyliques tels que la polyvinyl pyrrolidone (PVP) de haut poids moléculaire (par exemple de 1 300 000 g.mol⁻¹ ; Kollidon® 90F de BASF PVP K90) ou la polyvinyl pyrrolidone de faible poids moléculaire (par exemple de 60 000 g.mol⁻¹ ; Kollidon® 30 de BASF PVP K30);
- des polyéthers hydrosolubles tels que le polyéthylène glycol (PEG) de haut poids moléculaire (par exemple PEG à 900 000 g.mol⁻¹) ou le polyéthylène glycol (PEG) de faible poids moléculaire (par exemple le PEG à 1500 g.mol⁻¹, également nommé PEG 1500), ou encore
- des dérivés cellulosiques tels que l'HydroxyPropyl Methyl Cellulose HPMC, par exemple l'HPMC E4M (par exemple connu sous le nom commercial de Methocel™ E4M de DOW WOLFF CELLULOSICS ou de Benecel™ E4M d'ASHLAND) ou l'Ethyl Cellulose (par exemple l'Ethocel™ de DOW WOLFF CELLULOSICS ou encore Surelease® Clear de COLORCON).

Une molécule de haut poids moléculaire est une molécule présentant une masse molaire supérieure ou égale 90 000 g.mol⁻¹, ou supérieure ou égale à 100 000 g.mol⁻¹, ou supérieure ou égale à 200 000 g.mol⁻¹ ou encore supérieure ou égale à 300 000 g.mol⁻¹. Par défaut, une molécule est considérée comme présentant un faible poids moléculaire lorsqu'elle présente une masse molaire strictement inférieure à 90 000 g.mol⁻¹.

Il convient de noter que le HANa entre dans la catégorie des molécules de « haut poids moléculaire ». En revanche, l'héparine et l'Ethyl Cellulose entrent dans la catégorie du « faible poids moléculaire ».

La préparation du produit hydrophile s'effectue en solution aqueuse. La concentration du produit hydrophile dans la solution peut être comprise entre 0,05% et 2%, avantageusement entre 0,1% et 2%, entre 0,2% et 2% ou encore entre 1 % et 2%.

Aller au-delà de 2% s'avère inutile pour obtenir une meilleure enduction. De plus, plus ce pourcentage est élevé et plus le coût de production augmente.

Les parties du dispositif traitées par voie plasma sont alors trempées dans la solution puis, égouttées et séchées.

On en déduit incidemment, du fait de cette opération de trempage, que l'intégralité de la surface interne 11' du corps 1' de la seringue et/ou le cas échéant, l'intégralité de la surface externe 33' du bouchon 3' sont enduites. En effet, toute la surface interne 11' du corps 1' de la seringue (sur la longueur L ; cf. figure 2) peut être en contact avec le bouchon 3'. De même, sur l'exemple de la figure 2, le bouchon 3' présente une forme dont l'ensemble de la surface externe 33' est destinée à être en contact avec le corps 1' de la seringue.

Cependant, la forme du bouchon 3' peut varier d'une seringue à l'autre. On trouve par exemple des bouchons comportant à la fois une ou plusieurs parties destinées à être au contact de la surface interne 11' du corps 1' et une ou plusieurs parties en retrait, qui ne sont généralement pas mises au contact de cette surface interne 11' du corps 1', même en cas de déformation de l'élastomère formant le bouchon 3'.

Le dispositif est alors prêt pour être testé sur l'installation expérimentale décrite à l'appui de la figure 2.

L'installation expérimentale 100 permet d'obtenir des courbes reliant la force mesurée par le capteur 104 en fonction du déplacement du poussoir dans le corps du dispositif.

La forme théorique d'une telle courbe est représentée sur la figure 3, dans le cas d'une seringue.

Entre le point P0 et le point P1, le déplacement du poussoir 2' commence. On note que la force nécessaire pour mettre en mouvement le poussoir augmente en raison du frottement du bouchon 3' sur le corps 1' de seringue 10'.

Le point P1 correspond à un pic local de la force mesurée par le capteur, qui est associée à la force nécessaire pour décoller/débloquer le bouchon de la paroi du corps de seringue.

Cette force est appelée force de décollement.

Dans le cadre de l'invention, on cherche avantageusement à diminuer cette force de décollement, par rapport à la force de décollement constatée avec une seringue de l'art antérieur (bouchon enduit de silicone).

Entre les points P1 et P2, la force diminue suite au décollement du bouchon (relaxation liée au décollement).

Entre les points P2 et P3, la force de poussée est monotone, mais pas nécessairement inférieure au pic local de force liée à la force de décollement. Cette force de poussée correspond à la force exercée pendant toute la poussée du poussoir 2' le long du corps 1' de la seringue 10', après que la phase de décollement du bouchon 3' est terminée.

Les points P2 et P3 sont utilisés pour calculer une force, dite force moyenne de poussée, exercée sur le poussoir entre ces deux points.

Le point P4 correspond à la mise en butée du bouchon 3' contre le fond du corps 1' de la seringue 10'.

La fin de la mesure est effectuée lorsque la force mesurée est de 90N (non représenté sur la figure 3 pour des raisons de commodités). Plus précisément, les essais sont arrêtés lorsque l'on mesure une force égale à 90% de la force maximale susceptible d'être mesurée par la cellule. Cette force maximale est en l'occurrence de 100N. Cela est cohérent avec la mise en butée du bouchon 3' avec le fond du corps 1' de la seringue 10'.

La forme de la courbe représentée sur la figure 3 est générique. Ainsi, cette forme serait également obtenue pour une pompe telle que représentée sur la figure 1(b).

Les tests 1 à 4 qui seront présentés ci-après sont réalisés dans les conditions suivantes : vitesse de déplacement de la traverse 102 constante, en l'occurrence de 40mm/min (vitesse typique pour une seringue) ; corps 1' de seringue 10' rempli d'eau pure ; essai mené sur toute la longueur L du corps 1' de la seringue 10'.

Le corps 1' de seringue 10' peut être réalisé en matériau polymère, tel qu'un copolymère cyclo-oléfine, le polyéthylène ou le polypropylène, ou en verre. Dans la cadre des tests 1 à 4 présentés ci-après pour la seringue, le corps 1' de seringue 10' est réalisé en verre.

Le bouchon 3' de la seringue 10' est généralement réalisé en matériau élastomère. L'élastomère utilisé pour le bouchon 3' peut être choisi parmi le polyisoprène, les caoutchoucs synthétiques butyle, chlorobutyle ou bromobutyle, le caoutchouc naturel ou des mélanges et copolymères de ces différentes matières. En l'occurrence, il s'agit de chlorobutyle dans les tests réalisés avec des seringues, présentés ci-après.

### TEST 1: Hyaluronate de sodium (HANa)

Trois familles de seringues avec différents traitements ont été testées :
- famille de seringue n°11: bouchon traité avec du silicone (référence de l'art antérieur) ;
- famille de seringue n° 12 : corps de seringue non traité et bouchon traité avec HANa (invention) ;
- famille de seringue n° 13: corps de seringue et bouchon tous deux traités avec HANa (invention).

Pour chaque famille de seringue, plusieurs essais ont été effectués afin d'obtenir des données moyennes, en l'occurrence cinq essais avec cinq seringues préparées de la même façon.

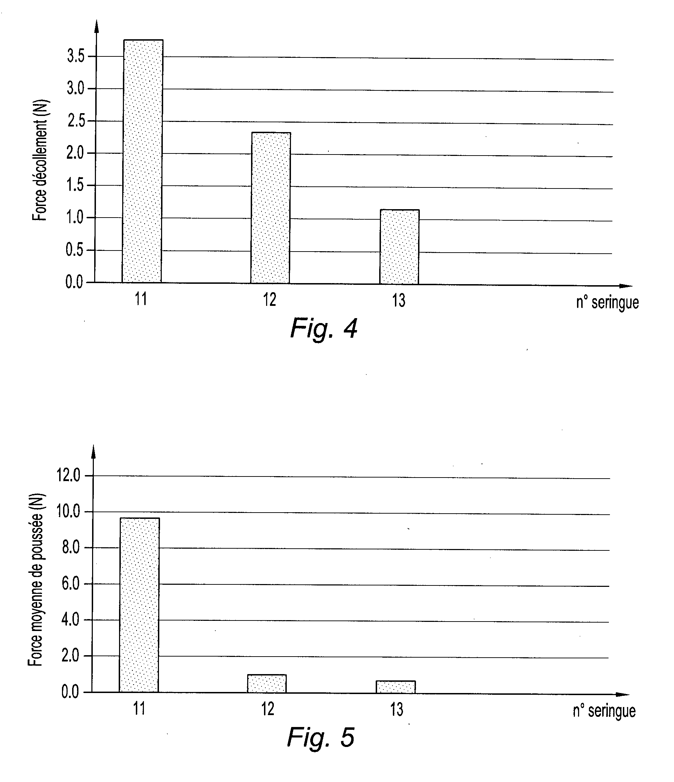

Les données fournies sur les figures 4 et 5 sont donc, pour chaque famille de seringue, des données moyennées sur ces cinq essais. Dans le cas particulier de la force moyenne de poussée, une première moyenne est donc effectuée entre les points P2 et P3 pour chaque essai, puis une seconde moyenne est effectuée entre les différents essais.

Chaque référence de famille de seringue est assimilée par la suite à une référence de seringue.

Par ailleurs, pour la préparation des seringues n°12 et n°13, la concentration de HANa dans la solution aqueuse était de 0,2%.

La figure 4 représente les résultats comparatifs obtenus pour ces différentes seringues sur la force de décollement.

Sur cette figure 4, on montre que les seringues 12 et 13 conformes à l'invention présentent une force de décollement plus faible que la force de décollement mesurée pour la seringue de référence (bouchon enduit de silicone).

En d'autres termes, ces seringues limitent le risque de blocage du bouchon 3' lors de sa mise en mouvement dans le corps 1' de la seringue.

Par ailleurs, il importe peu que le traitement avec le HANa soit effectué sur le bouchon 3' seul ou à la fois sur le corps 1' de la seringue 10' et le bouchon 3'.

Cependant, on note qu'une seringue pour laquelle le bouchon 3' et le corps 1' ont été enduits de HANa (seringue n° 13) présente une force de décollement plus faible qu'une seringue pour laquelle seul le bouchon 3' a été enduit de HANa (seringue n°12).

En définitive, la solution qui exige la force de décollement la plus faible consiste à enduire à la fois le bouchon 3' et le corps 1' de la seringue 10' avec du HANa.

La figure 5 représente les résultats comparatifs obtenus pour les seringues n°11 (référence), n°12 et n°13 sur la force moyenne de poussée exercée par le poussoir 2' dans son mouvement le long du corps 1', après décollement du bouchon 3'.

Là également, on montre une nette diminution de la force moyenne de poussée exercée par le poussoir 2' dans son mouvement le long du corps 1' avec les seringues n° 12 et n° 13 conformes à l'invention, par rapport à celle qui est mesurée avec la seringue de référence (bouchon enduit de silicone).

Cependant, on note qu'une seringue pour laquelle le bouchon 3' et le corps 1' ont été enduits de HANa (seringue n° 13) présente une force moyenne de poussée encore plus faible que celle obtenue avec une seringue avec bouchon traité au silicone pour laquelle seul le bouchon 4' a été enduit de HANa (seringue n°12).

Les seringues selon l'invention, enduites de HANa, présentent donc des caractéristiques de glissement améliorées par rapport à celles dont le bouchon est enduit de silicone.

La seringue n°13 est cependant une solution particulièrement avantageuse, tant sur le plan de la force de décollement que de la force moyenne de poussée.

### TEST 2: polyéthylène glycol, 900 000g/mol (PEG 900000)

Trois familles de seringue avec différents traitements ont été testées :
- famille de seringue n° 21: bouchon traité avec du silicone (référence de l'art antérieur) ;
- famille de seringue n° 22 : corps de seringue non traité et bouchon traité avec PEG 900000 (invention) ;
- famille de seringue n° 23 : corps de seringue et bouchon tous deux traités avec PEG 900000 (invention).

Pour chaque famille de seringues, plusieurs essais ont été effectués afin d'obtenir des données moyennes, en l'occurrence 10 essais avec 10 seringues préparées de la même façon.

Par la suite, chaque référence de famille est assimilée à une seringue de même référence.

Pour la préparation des seringues n°22 et n°23, la concentration de PEG dans la solution aqueuse était de 0,2%.

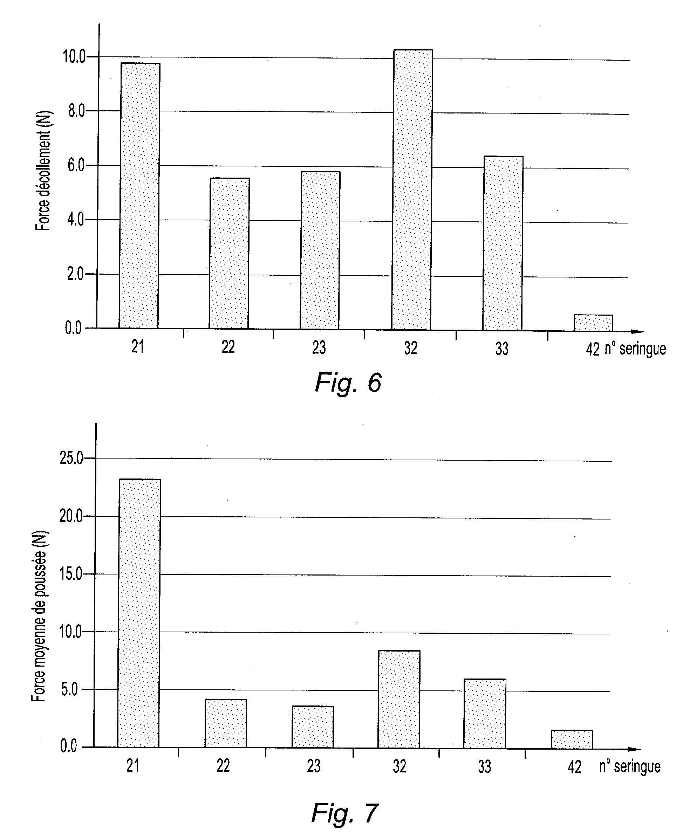

Les résultats de ces essais sont représentés sur la figure 6 pour la force de décollement et, sur la figure 7 pour la force moyenne de poussée.

On montre que la seringue n° 22 présente une force de décollement plus faible que celle qui est obtenue avec la seringue de référence (bouchon enduit de silicone). On montre également que la force moyenne de poussée est bien plus faible avec cette seringue n° 22 que celle qui est obtenue avec la seringue de référence.

Des remarques similaires peuvent être effectuées en comparant la seringue n°23 à la seringue de référence.

Par ailleurs, lorsque l'on compare les seringues n°22 et n°23 conformes à l'invention entre elles, il apparaît qu'elles offrent des performances comparables, tant sur le plan de la force de décollement que de la force moyenne de poussée.

### TEST 3: PolyVinvl Pyrrolidone K90 (PVP K90)

Trois familles de seringue avec différents traitements ont été testées :
- famille de seringue n°21: bouchon traité avec du silicone (référence de l'art antérieur) ;
- famille de seringue n° 32 : corps de seringue non traité et bouchon traité avec PVP K90 (invention) ;
- famille de seringue n° 33 : corps de seringue et bouchon tous deux traités avec PVP K90 (invention).

Pour chaque famille de seringues, plusieurs essais ont été effectués afin d'obtenir des données moyennes, en l'occurrence 10 essais avec 10 seringues préparées de la même façon.

Par la suite, chaque référence de famille est assimilée à une seringue de même référence.

Pour la préparation des seringues n°32 et n°33, la concentration de PVP K90 dans la solution aqueuse était de 0,2%.

Les résultats de ces essais sont représentés sur la figure 6 pour la force de décollement et, sur la figure 7 pour la force moyenne de poussée.

On montre que la seringue n° 32 présente une force de décollement similaire à celle qui est obtenue avec la seringue de référence (bouchon enduit de silicone). On montre également que la force moyenne de poussée obtenue avec cette seringue n°32 est bien plus faible que celle qui est obtenue avec la seringue de référence.

On montre également que la seringue n° 33 présente une force de décollement bien plus faible que celle qui est obtenue avec la seringue de référence. Par ailleurs, on montre que la force moyenne de poussée est également bien plus faible que celle qui est obtenue avec la seringue de référence.

Lorsque l'on compare les seringues n°32 et n°33 conformes à l'invention entre elles, une seringue dont le bouchon et le corps sont tous deux enduits de PVP K90 est plus avantageuse, tant sur le plan de la force de décollement que sur le plan de la force moyenne de poussée.

Il convient de noter que les TEST2 et TEST 3 ont été effectués en même temps, ce qui explique pourquoi la référence choisie (seringue n°21) est la même pour ces deux tests.

### TEST 4 : HydroxylPropyl Methyl Cellulose (HPMC E4M)

Les inventeurs ont pu tester une seringue avec une enduction soit du bouchon seul, soit à la fois du bouchon et du corps de la seringue, par du HPMC E4M. Pour ces tests, la concentration de HPMC E4M dans la solution aqueuse était de 0,2%. Ces tests montrent des performances similaires à celles d'une seringue de référence (bouchon enduit de silicone) quant à la force de décollement et des performances meilleures quant à la force moyenne de poussée.

Ces tests ont été complétés par un autre test dans le cas où seul le bouchon est traité avec du HPMC E4M, la concentration de HPMC E4M dans la solution aqueuse étant cette fois-ci de 1%.

Deux familles de seringues avec différents traitements ont été testées :
- famille de seringue n°21 : bouchon traité avec du silicone (référence de l'art antérieur) ;
- famille de seringue n°42 : corps de seringue non traité et bouchon traité avec du HPMC E4M (invention).

Pour chaque famille de seringues, plusieurs essais ont été effectués afin d'obtenir des données moyennes, en l'occurrence 20 essais avec 20 seringues préparées de la même façon.

Par la suite, chaque famille de seringues est assimilée à une seringue de même référence.

Pour la préparation des seringues n°42, la concentration d'HPMC E4M dans la solution aqueuse était, comme indiqué ci-dessus, de 1%.

Les résultats de ces essais sont présentés sur la figure 6 pour la force de décollement et, sur la figure 7 pour la force moyenne de poussée.

On montre que la seringue n°42 présente une force de décollement plus faible que celle qui est obtenue avec la seringue de référence (seringue n°21). On montre également que la force moyenne de poussée est bien plus faible avec cette seringue n°42 que celle qui est obtenue avec la seringue de référence.

Une seringue dont le corps est traité avec du HANa et dont le bouchon ne serait pas traité n'a pas été testée, car il s'agit d'une solution intermédiaire entre les deux solutions extrêmes correspondant aux seringues n°12 (seul le bouchon 3' est traité) et n°13 (le corps 1' et le bouchon 3' sont tous deux traités). En effet, l'enduction du seul corps de seringue s'effectue sur une surface plus importante que celle du seul bouchon, mais moins importante que celle de l'ensemble formé par le corps de la seringue et le bouchon.

Et compte tenu des résultats présentés sur les figures 4 et 5, une telle seringue présenterait les mêmes avantages, vis-à-vis de la seringue de référence (bouchon enduit de silicone), que les seringues n°12 et n°13.

Des remarques similaires peuvent être effectuées dans l'hypothèse où le HANa est remplacé par le PEG 900000, par le PVPK90 ou par le HPMC E4M.

Enfin, parmi les différentes molécules que les inventeurs ont identifié, il apparaît que la solution la plus intéressante, en termes de performances par rapport à l'art antérieur, consiste à enduire à la fois le bouchon et le corps de la seringue avec du HANa. En effet, c'est cette molécule qui permet d'obtenir une diminution de la force de décollement et de la force moyenne de poussée la plus significative pour une concentration dans la solution aqueuse identique (comparaison possible pour une concentration de 0,2% sur les tests 1 à 3, donc hors test complémentaire sur le test 4).

Le test complémentaire (seringue n°42), réalisé avec du HPMC E4M, montre que ce produit permet également de résoudre le problème posé par l'invention et, peut être, que l'augmentation de la concentration du produit hydrophile dans la solution aqueuse (passage de 0,2% à 1%) peut avoir un impact sur l'amélioration de la performance de la seringue (diminution de la force de décollement ; diminution de la force moyenne de poussée).

D'autres tests (TESTS 5.1 à 5.4) ont été réalisés avec des seringues.

Sauf indication contraire mentionnée ci-après, les conditions de tests et les seringues sont les mêmes que celles décrites précédemment.

Pour ces autres tests, la vitesse de la traverse 102, a été fixée à 100 mm/min.

Par ailleurs, le corps 1' de seringue 10' est réalisé en verre.

L'objectif de ces tests est de mettre en évidence que certaines molécules ne sont pas intéressantes pour l'objectif recherché.

Pour l'ensemble de ces tests, la famille de seringue n° 50 pour laquelle aucun traitement n'est prévu sert de référence (témoin négatif). Elle présente donc, comme cela est connu de l'homme du métier des propriétés de glissement plus faibles qu'une seringue qui serait traitée au silicone, cette dernière ayant notamment servi de référence (témoin positif) dans les tests 1 à 4. Autrement dit, lorsqu'une seringue présente des propriétés de glissement moins bonnes que la seringue de référence n°50, l'homme du métier peut logiquement s'attendre à ce que ses propriétés de glissement soient également moins intéressantes qu'une seringue siliconée.

Pour ces autres tests, plusieurs essais ont été effectués afin d'obtenir des données moyennes, en l'occurrence 20 essais avec 20 seringues préparées de la même façon.

### TEST 5.1: au sein de la famille des Glycosaminoglycanes; comparaison entre le HANa et l'héparine.

- famille de seringue n° 514 : bouchon non traité et corps de seringue traité et avec HANa (invention) ;
- famille de seringue n° 515 : bouchon non traité et corps de seringue traité avec de l'héparine (hors invention).

Pour la préparation des seringues n°514 et n°515, la concentration de HANa dans la solution aqueuse était de 0,1%.

### TEST 5.2 : au sein de la famille des polymères vinyliques (hydrosolubles) ; comparaison entre la PVP K90 et la PVP K30.

- famille de seringue n° 524 : bouchon non traité et corps de seringue traité et avec la PVP K90 (invention) ;
- famille de seringue n° 525 : bouchon non traité et corps de seringue traité avec la PVP K30 (hors invention).

Pour la préparation des seringues n°524 et n°525, la concentration de PVP, K90 ou K30 selon le cas, dans la solution aqueuse était de 1%.

### TEST 5.3 : au sein de la famille des polyéthers hydrosolubles ; comparaison entre PEG 900 000 le PEG 1500.

- famille de seringue n° 534 : bouchon non traité et corps de seringue traité et avec la PEG 900 000 (invention) ;
- famille de seringue n° 535 : bouchon non traité et corps de seringue traité avec la PEG 1500 (hors invention).

Pour la préparation des seringues n°534 et n°535, la concentration de PEG, 900 000 ou 1500 selon le cas, dans la solution aqueuse était de 1%.

### TEST 5.4 : au sein de la famille des dérivés cellulosiques ; comparaison entre l'HPMC E4M et l'Ethyl Cellulose (EC).

- famille de seringue n° 544 : bouchon non traité et corps de seringue traité et avec l'HPMC E4M (invention) ;
- famille de seringue n° 545 : bouchon non traité et corps de seringue traité avec l'Ethyl Cellulose ou EC (hors invention).

Pour la préparation des seringues n°544 et n°545, la concentration du dérivé cellulosique dans la solution aqueuse était de 1%.

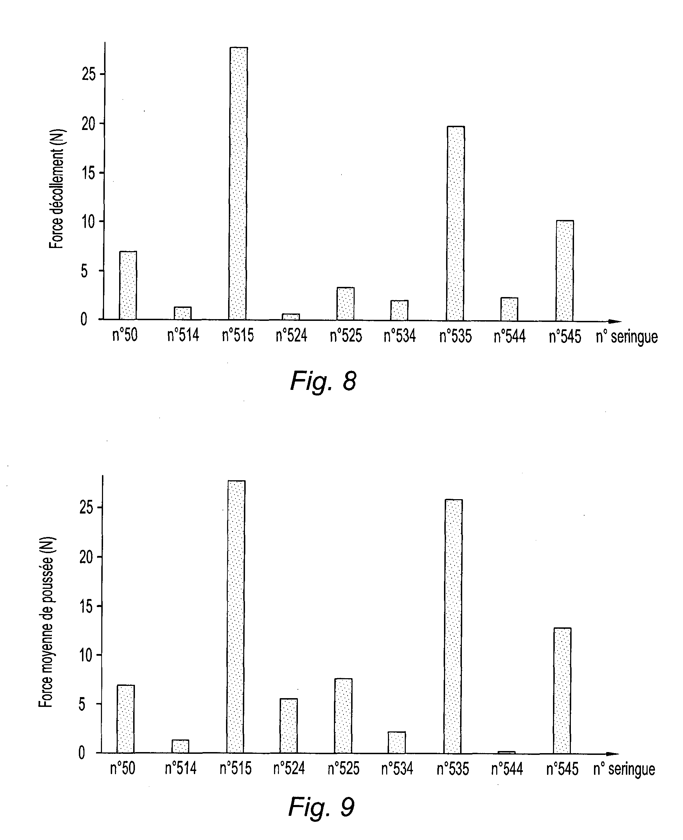

Les résultats obtenus pour ces différents tests sont représentés sur la figure 8, en ce qui concerne la force de décollement, et sur la figure 9, en ce qui concerne la force moyenne de poussée.

On note que l'héparine (n°515), la PVP K30 (n° 525), le PEG 1500 (n°535) et l'EC (n°545) ne permettent pas d'obtenir des propriétés de glissement intéressantes, tant sur le plan de la force de décollement que sur le plan de la force moyenne de poussée le long du corps de seringue.

Pour les autres familles de seringues, à savoir, le HANa (n°514), la PVP K90 (n°524), le PEG 900 000 (n°534) et le HPMC E4M (n°544), les résultats obtenus sont conformes à ceux présentés dans les conditions des tests 1 à 4, pour des vitesses de déplacement du bouchon par rapport au corps de pompe plus élevées (100mm/mn pour les TESTS 5.1 à 5.4 ; 40mm/mn pour les TESTS 1 à 4).

Cela montre que la vitesse de poussée du bouchon 3' ne change pas qualitativement les résultats obtenus.

De plus, il convient de noter que pour une pompe, telle que celle représentée sur la figure 1(b), la vitesse de déplacement du bouchon par rapport au corps est généralement plus proche de 100 mm/mn que de 40 mm/mn (car il y a généralement une aide pour la propulsion du produit à délivrer, tel que de l'air sous pression). De ce fait, les résultats obtenus pour la seringue sont transposables au cas de la pompe.

Nous avons parlé précédemment de l'application à la seringue et à la pompe.

Toutefois, l'invention concerne plus généralement tout dispositif comprenant au moins un corps, un poussoir et un bouchon situé à l'extrémité du poussoir pour assurer l'étanchéité entre le poussoir et le corps, caractérisé en ce qu'au moins l'un, d'une part, de l'intégralité de la surface interne du corps ou, d'autre part, d'au moins une partie de la surface externe du bouchon, est enduite d'un produit hydrophile choisi parmi le hyaluronate de sodium (HANa), la polyvinylpyrrolidone (PVP) de haut poids moléculaire, le polyéthylène glycol (PEG) de haut poids moléculaire ou l'HydroxyPropyl Methyl Cellulose (HPMC).

Ce dispositif est un dispositif de diffusion de produit fluide, notamment à visée médicamenteuse.

En particulier, ce dispositif peut être tout dispositif comprenant au moins un corps, un poussoir et un bouchon situé à l'extrémité du poussoir pour assurer l'étanchéité entre le poussoir et le corps, à l'exception d'une seringue, caractérisé en ce qu'au moins l'un, d'une part, de l'intégralité de la surface interne du corps ou, d'autre part, d'au moins une partie de la surface externe du bouchon, est enduite d'un produit hydrophile choisi parmi le hyaluronate de sodium (HANa), la polyvinylpyrrolidone (PVP) de haut poids moléculaire, le polyéthylène glycol (PEG) de haut poids moléculaire ou l'HydroxyPropyl Methyl Cellulose (HPMC).

En variante, ce dispositif peut être une seringue.

## Revendications

1. Dispositif comprenant au moins un corps (1'), un poussoir (2') et un bouchon (3') situé à l'extrémité du poussoir pour assurer l'étanchéité entre le poussoir et le corps, **caractérisé en ce que** l'un au moins, d'une part, de l'intégralité de la surface interne (11') du corps ou, d'autre part, d'au moins une partie de la surface externe (33') du bouchon (3'), est enduite d'un produit hydrophile choisi parmi le Hyaluronate de sodium (HANa), la polyvinylpyrrolidone (PVP) de haut poids moléculaire, le polyéthylène glycol (PEG) de haut poids moléculaire ou l'Hydroxypropyl Methyl Cellulose (HPMC).

2. Dispositif selon la revendication 1, **caractérisé en ce que** l'intégralité de la surface interne (11') du corps (1') et au moins une partie de la surface externe (33') du bouchon (3') sont enduites d'un même produit hydrophile.

3. Dispositif selon l'une des revendications précédentes, **caractérisé en ce que** l'intégralité de la surface externe (33') du bouchon (3') est enduite du produit hydrophile.

4. Dispositif selon l'une des revendications précédentes, **caractérisé en ce que** la polyvinylpyrrolidone de haut poids moléculaire est la PVP K90.

5. Dispositif selon l'une des revendications précédentes, **caractérisé en ce que** le polyéthylène glycol de haut poids moléculaire est le PEG 900 000.

6. Dispositif selon l'une des revendications précédentes, **caractérisé en ce que** l'HPMC est l'HPMC E4M.

7. Dispositif selon l'une des revendications précédentes, dans lequel le corps (1') est réalisé en verre ou en polymère tel qu'un copolymère cyclo-oléfine, le polyéthylène ou le polypropylène.

8. Dispositif selon l'une des revendications précédentes, à l'exception d'une seringue.

9. Dispositif selon l'une des revendications précédentes, **caractérisé en ce qu'**il s'agit d'une pompe.

10. Dispositif selon l'une des revendications 1 à 7, **caractérisé en ce qu'**il s'agit d'une seringue.

11. Dispositif selon la revendication précédente, **caractérisé en ce que** le bouchon (3') est réalisé en élastomère.

## Patentansprüche

1. Vorrichtung, umfassend mindestens einen Körper (1'), einen Schieber (2') und eine Kappe (3'), die sich am Ende des Schiebers befindet, um die Dichtigkeit zwischen dem Schieber und dem Körper zu sichern, **dadurch gekennzeichnet, dass** zum einen mindestens entweder die Gesamtheit der inneren Fläche (11') des Körpers oder zum anderen mindestens ein Teil der äußeren Fläche (33') der Kappe (3') mit einem hydrophilen Produkt beschichtet ist, das aus dem Natriumhyaluronat (HANa), dem Polyvinylpyrrolidon (PVP) mit hohem Molekulargewicht, dem Polyethylenglycol (PEG) mit hohem Molekulargewicht oder der Hydroxypropylmethylcellulose (HPMC) ausgewählt ist.

2. Vorrichtung nach Anspruch 1, **dadurch gekennzeichnet, dass** die Gesamtheit der inneren Fläche (11') des Körpers (1') und mindestens ein Teil der äußeren Fläche (33') der Kappe (3') mit einem selben hydrophilen Produkt beschichtet sind.

3. Vorrichtung nach einem der vorangehenden Ansprüche, **dadurch gekennzeichnet, dass** Gesamtheit der äußeren Fläche (33') der Kappe (3') mit dem hydrophilen Produkt beschichtet ist.

4. Vorrichtung nach einem der vorangehenden Ansprüche, **dadurch gekennzeichnet, dass** das Polyvinylpyrrolidon mit hohem Molekulargewicht das PVP K90 ist.

5. Vorrichtung nach einem der vorangehenden Ansprüche, **dadurch gekennzeichnet, dass** das Polyethylenglycol mit hohem Molekulargewicht das PEG 900 000 ist.

6. Vorrichtung nach einem der vorangehenden Ansprüche, **dadurch gekennzeichnet, dass** das HPMC das HPMC E4M ist.

7. Vorrichtung nach einem der vorangehenden Ansprüche, wobei der Körper (1') aus Glas oder aus Polymer wie ein Cycloolefin-Copolymer, das Polyethylen oder das Polypropylen hergestellt ist.

8. Vorrichtung nach einem der vorangehenden Ansprüche, mit Ausnahme einer Spritze.

9. Vorrichtung nach einem der vorangehenden Ansprüche, **dadurch gekennzeichnet, dass** es sich um eine Pumpe handelt.

10. Vorrichtung nach einem der Ansprüche 1 bis 7, **dadurch gekennzeichnet, dass** es sich um eine Spritze handelt.

11. Vorrichtung nach vorangehendem Anspruch, **dadurch gekennzeichnet, dass** die Kappe (3') aus Elastomer hergestellt ist.

## Claims

1. A device comprising at least a body (1'), a pusher (2'), and a plunger (3') situated at the end of the pusher for providing sealing between the pusher and the body, the device being **characterized in that** at least one firstly of the entire inside surface (11') of the body and secondly of at least a portion of the outside surface (33') of the plunger (3') is coated in a hydrophilic substance selected from sodium hyaluronate (HANa), polyvinyl pyrrolidone (PVP) of high molecular weight, polyethylene glycol (PEG) of high molecular weight, and hydroxypropyl methyl cellulose (HPMC).

2. A device according to claim 1, **characterized in that** the entire inside surface (11') of the body (1') and at least a portion of the outside surface (33') of the plunger (3') are coated in the same hydrophilic substance.

3. A device according to either preceding claim, **characterized in that** the entire outside surface (33') of the plunger (3') is coated in the hydrophilic substance.

4. A device according to any preceding claim, **characterized in that** the polyvinyl pyrrolidone of high molecular weight is PVP K90.

5. A device according to any preceding claim, **characterized in that** the polyethylene glycol of high molecular weight is PEG 900,000.

6. A device according to any preceding claim, **characterized in that** the HMPC is HMPC E4M.

7. A device according to any preceding claim, wherein the body (1') is made of glass or of a polymer such as a cyclic olefin copolymer, polyethylene, or polypropylene.

8. A device according to any preceding claim, with the exception of a syringe.

9. A device according to any preceding claim, **characterized in that** it is a pump.

10. A device according to any one of claims 1 to 7, **characterized in that** it is a syringe.

11. A device according to the preceding claim, **characterized in that** the plunger (3') is made of elastomer.
